# FASCICULE DE BREVET EUROPEEN

(11) **EP 3 372 313 B1**
(45) Date de publication et mention de la délivrance du brevet: **19.06.2019**
(21) Numéro de dépôt: 18154251.5
(22) Date de dépôt: 30.01.2018
(51) Int. Cl.: B01L 9/00

(54) **PLATEAU DE STOCKAGE DE LAMES D'ÉCHANTILLONNAGE, MEUBLE POUR SON RANGEMENT ET DISPOSITIF D'IDENTIFICATION DESDITES LAMES**
LAGERTABLETT FÜR MUSTERKLINGEN, MÖBELSTÜCK FÜR DESSEN AUFNAHME UND VORRICHTUNG ZUR IDENTIFIZIERUNG DIESER KLINGEN
TRAY FOR STORING SAMPLE PLATES, CABINET FOR STORING SAME AND DEVICE FOR IDENTIFYING SAID PLATES

(30) Priorité: 07.03.2017 FR 1751817; 13.06.2017 FR 1755307
(43) Date de publication de la demande: 12.09.2018
(73) Titulaire: Dreampath Diagnostics, 67100 Strasbourg (FR)
(72) Inventeur: JORDAN NAVAS, Pablo, BRISTOL, BS83DG (GB); WILHELM, Valérie, 67114 ESCHAU (FR); CREMEL, Arnaud, 67114 ESCHAU (FR)
(74) Mandataire: Cabinet Bleger-Rhein-Poupon

(56) Documents cités:
- EP-A1- 2 883 813
- EP-A2- 1 380 345
- WO-A1-2006/098441
- WO-A1-2010/004331
- US-A1- 2002 183 882

## Description

La présente invention entre dans le domaine médical de l'analyse de prélèvements tissulaires et cellulaires.

L'invention concerne plus particulièrement la conservation et le stockage ordonnés de tels prélèvements.

Dans le cadre de la prise en charge médicale d'un patient ou dans le cadre de la recherche, humaine ou animale ou végétale, des prélèvements tissulaires ou cellulaires peuvent être effectués et échantillonnés en vue d'une analyse histologique et/ou moléculaire. A des fins d'observation et de conservation, ces prélèvements sont disposés, généralement au centre, sur un support se présentant sous la forme d'une lame rectangulaire en matériau transparent, en matériau plastique mais plus souvent en verre transparent, notamment en «verre blanc ». De telles lames présentent des dimensions standardisées, généralement de 75 à 76 mm (millimètres) de longueur, pour 25 à 26 mm de largeur, pour une épaisseur d'environ 1 mm. Ces dimensions sont particulièrement adaptées à une utilisation en analyse microscopique.

Un traitement peut être appliqué à l'échantillon ainsi disposé sur une telle lame, comme par exemple une coloration, avant d'être recouvert par une lamelle, généralement de forme carrée, rectangulaire ou ronde, ou un film transparent. Un matériau adhésif protecteur peut aussi être ajouté supérieurement pour lier la lame et la lamelle, assurant la conservation dans le temps de l'échantillon et permettant la lecture au microscope. Enfin, des données relatives à l'échantillon et à son patient sont appliquées sur une partie latérale de la lame, en vue de son classement et de son identification ultérieure. Ces données peuvent être directement apposées sur la partie de la lame comportant une portion prévue à cet effet, ou bien sous forme d'une étiquette adhésive. L'ensemble forme ainsi une lame échantillonnée.

L'invention concerne le stockage ordonné de telles lames échantillonnées, la conservation des lames stockées de manière ordonnée, ainsi que la gestion informatique du stockage de ces lames échantillonnées.

A cet effet, l'invention concerne un plateau de stockage et d'ordonnancement de lames échantillonnées, un meuble conformé pour héberger au moins un plateau de stockage de lames échantillonnées, ainsi qu'un dispositif spécifique permettant une identification desdites lames échantillonnées contenues au sein d'un tel plateau de stockage.

Il convient de noter qu'au sens de la présente invention, sauf stipulé autrement les termes « lames » et « lames échantillonnées » sont équivalents.

Actuellement, une fois préparées, les lames échantillonnées sont rangées et manipulées manuellement, par des opérateurs n'ayant pas forcément reçu de formation spécifique, sans aucun contrôle. De ce fait, cette gestion manuelle précaire entraîne des erreurs et une perte de temps considérable, pouvant aller jusqu'à l'égarement préjudiciable de certaines lames.

Plus précisément, en vue de leur stockage, les lames échantillonnées sont superposées par ordre alpha numérique en piles pouvant aller jusqu'à plusieurs centaines. Lesdites piles sont ensuite placées au sein d'un rangement, tel une boîte ou un tiroir. Une fois rangée, il est alors difficile de retrouver une lame en particulier au sein de sa pile, étant donné que ses informations d'identification sont cachées par les autres lames empilées, à l'exception éventuellement de la lame se trouvant sur le dessus de chaque pile.

De façon connexe, les manipulations des lames empilées génèrent souvent un raclement susceptible d'endommager la matière biologique qui s'y trouve déposée.

En l'état, il existe un réel problème lié au rangement ordonné des lames échantillonnées en vue d'assurer un classement et une gestion permettant ultérieurement de retrouver rapidement, simplement, et de façon sûre, l'endroit où est stockée une lame particulière.

Une solution à ce problème, proposée au travers du document WO 2010/004331, consiste en un dispositif pourvu de tiroirs au sein desquels sont positionnées verticalement des lames échantillonnées pourvues de moyens d'identification. En d'autres termes, le tiroir s'étend selon un plan horizontal ou sensiblement horizontal tandis que les lames sont disposées orthogonalement par rapport à un tel plan horizontal.

Plus précisément, chaque tiroir est compartimenté et présente une série de fourreaux individuels alignés, régulièrement espacés sur sa longueur, à l'intérieur desquels lesdites lames peuvent être glissées par le haut, les étiquettes d'identification dont elles sont pourvues étant orientées vers le haut. De plus, chaque tiroir est monté coulissant, au travers d'une ouverture, sur un cadre support équipé supérieurement d'un lecteur radio de type RFID (pour « Radio Frequency Identification ») permettant de lire à distance les informations enregistrées au sein de puces contenues dans lesdites étiquettes adhésives apposées sur chacune des lames.

Ainsi, lors de chaque fermeture du tiroir, les informations contenues dans les étiquettes respectives des lames qui y sont contenues, sont lues par le lecteur, ce qui permet d'identifier chacune desdites lames et d'enregistrer leur position respective au sein du tiroir.

Un problème majeur, lié à l'utilisation de la technologie RFID, résulte dans le champ d'émission du lecteur, qui même localisé, envoie des ondes radio susceptibles d'entrer en communication avec les puces de plusieurs lames positionnées de façon adjacente au sein d'un même tiroir. De plus, la vitesse de translation du tiroir actionné manuellement, influe sur la qualité de lecture effectuée par le lecteur. De surcroît, si un emplacement est vide, le lecteur n'est pas à même de détecter l'absence de lame et identifiera celle contenue dans l'emplacement suivant, ce qui génère une incertitude quant aux positions des lames au sein du tiroir. Enfin, outre le fait que l'appareil objet de WO 2010/004331 ne permet pas toujours de déterminer automatiquement la position exacte d'une lame recherchée, la disposition verticale de plusieurs lames de façon adjacente, avec un moindre espace entre elles, ne permet pas à un opérateur de retrouver visuellement, de façon simple et rapide, ladite lame recherchée ainsi stockée, l'étiquette de la lame située à l'avant cachant celle de la lame située derrière. Il a par ailleurs été constaté que la technologie employée, reposant sur des puces RFID de durée de vie limitée, n'est pas toujours adaptée pour garantir l'identification, au cours du temps, de lames échantillonnées dont la durée moyenne de stockage est de plusieurs dizaines d'années.

Une solution alternative, consistant à positionner les lames horizontalement, dans des logements ménagés au sein d'un support sous forme de plateau, a également été proposée. Les logements se présentent sous la forme d'alvéoles formant des creux de forme parallélépipédique rectangle et de dimensions en général au moins partiellement complémentaires à celles desdites lames, afin de les recevoir en insertion par le dessus. Les lames se retrouvent donc positionnées à plat au sein de la face supérieure ouverte d'un tel plateau, disposées les unes à côté des autres selon des colonnes et des rangées, sans se chevaucher, dans un plan parallèle au fond dudit plateau. Cette disposition permet de lire aisément, de façon visuelle ou automatisée, leurs données d'identification respectives alors tournées vers le haut, et non masquées par les éventuelles lames adjacentes.

Toutefois, une telle disposition des lames sur un tel plateau, nécessite un espace accru de stockage, au regard d'un empilement ou bien d'une disposition dans lesquelles elles s'étendent les unes à côté des autres, perpendiculairement au fond d'un tiroir. En effet, une disposition à plat des lames juxtaposées nécessite une surface de stockage équivalente à la somme de la superficie desdites lames. Le nombre de lames pouvant être ainsi stockées sur un seul et même plateau est par conséquent limité, et correspond à une dimension de plateau maximale au-delà de laquelle ce dernier s'avère trop encombrant et difficile à manipuler. Ainsi, avec une telle solution, afin de stocker un grand nombre de lames, il est nécessaire de répartir les lames sur plusieurs plateaux, de dimensions acceptables en termes de manutention et d'encombrement. Toutefois, il est alors nécessaire d'effectuer également une identification de chaque plateau pour trouver une lame recherchée.

En outre, une disposition des lames parallèlement au fond d'un tel plateau, simplement enfoncées à plat au sein d'alvéoles ouvertes supérieurement, bien que de forme et de dimension ajustées à celles des lames, entraîne un maintien plus que précaire de ces dernières. Un tel plateau étant destiné à être déplacé et manipulé, un risque non négligeable subsiste que les lames glissent hors de leur alvéole en cas d'inclinaison intempestive dudit plateau, notamment s'il se retourne ou se renverse en raison de l'inadvertance de la personne qui le manipule.

Une solution à ce problème a consisté à adapter la structure des alvéoles destinées à recevoir les lames, par exemple en les complétant de redents ou bossages, aboutissant à une insertion en force de chaque lame dans l'alvéole considérée. Toutefois, en contrepartie, il devient alors difficile d'extraire une lame de son alvéole, en raison de ce maintien excessif, occasionnant une perte de temps, allant parfois jusqu'à la détérioration, voire la casse de la lame lors d'une extraction quelque peu forcée.

Il existe donc un réel problème quant au maintien des lames au sein de leur alvéole, qui doit être tel qu'elles n'en soient pas délogées de manière intempestive, tout en permettant des opérations d'insertion et d'extraction aisées et rapides, sans risque de détérioration des lames.

La présente invention a pour but de pallier les inconvénients de l'état de la technique en proposant une solution, pour la conservation et le stockage ordonné de lames échantillonnées, améliorée par rapport à celles de l'état de la technique. En particulier, l'invention vise à proposer une solution permettant de ranger lesdites lames de manière telle qu'elles puissent être identifiées et localisées de façon certaine, et d'enregistrer l'ensemble des données correspondantes au sein d'un système de référencement pour pouvoir les retrouver rapidement et simplement lorsqu'elles sont recherchées.

De plus, l'invention a pour but de proposer une solution de rangement avec laquelle le nombre de lames stockées est optimisé par rapport à l'encombrement généré, et qui garantisse une parfaite visualisation des données d'identification de chacune des lames ainsi que la lecture de l'ensemble d'entre elles.

Ainsi, l'invention a pour objet un plateau destiné au stockage de lames d'échantillonnage, constitué d'une plaque comprenant une pluralité d'alvéoles alignées, ménagées en creux au sein de l'épaisseur de ladite plaque, selon une forme et des dimensions conçues aptes à épouser au moins partiellement lesdites lames, chaque alvéole étant au moins constituée par un fond et deux parois latérales opposées, présentant une ouverture d'entrée et étant au moins en partie fermée par une paroi transversale s'étendant entre les parois latérales opposées, en face de ladite ouverture d'entrée, chaque alvéole comprenant au moins deux redents respectivement ménagés en saillie au niveau de chacune des parois latérales, lesdits redents s'étendant depuis l'ouverture d'entrée jusqu'à la paroi transversale, parallèlement au fond et formant avec les redents des alvéoles adjacentes des coulisses destinées à permettre un guidage en translation des bords d'une lame introduite dans une alvéole, un tel plateau étant caractérisé par le fait que le fond de chaque alvéole est prévu incliné d'un angle α par rapport au plan dudit plateau tandis que la longueur de chaque coulisse est inférieure à la longueur d'une lame destinée à être stockée dans une alvéole.

En fait, l'invention prévoit ainsi une disposition spécifique des lames échantillonnées, à savoir ni verticalement ni horizontalement par rapport au plan contenant le plateau, mais de façon inclinée par rapport à celui-ci. Ainsi, les alvéoles que comporte le plateau selon l'invention sont spécifiquement conformées pour recevoir les lames en insertion, et de manière telle qu'elles soient inclinées par rapport au plan dans lequel s'étend ledit plateau. Cette inclinaison permet une superposition partielle desdites lames. De fait, les alvéoles sont avantageusement dimensionnées de manière telle que lorsque les lames y sont logées, une portion d'une première lame est recouverte par une autre lame adjacente, tandis qu'une autre portion de ladite première lame, comportant les données d'identification, n'est pas recouverte. En somme, chaque lame dépasse de son alvéole, pour recouvrir une partie de la lame insérée dans l'alvéole située en dessous mais laisser cependant découverte la partie de cette lame comportant les informations d'identification. Ce recouvrement partiel permet de diminuer considérablement la surface du plateau pour un même nombre de lames stockées. En contrepartie, son épaisseur est augmentée en fonction de l'angle d'inclinaison du fond des alvéoles, mais de façon moindre, étant donné l'épaisseur minime des lames au regard de leur longueur.

L'invention permet donc d'obtenir un stockage sur une moindre surface d'un plus grand nombre de lames, par rapport à la surface occupée par une disposition connue à plat juxtaposée des lames.

En outre, cette disposition en recouvrement partiel, laissant visible les données apposées sur chaque lame, autorise, d'une part, une localisation précise et, d'autre part, une reconnaissance visuelle ou automatisée aisée et infaillible de chaque alvéole ainsi que de chaque lame qu'elle contient.

Conformément à l'invention, l'angle d'inclinaison α du fond de chaque alvéole est compris entre 5° et 45°, de préférence entre 8° et 25°.

Pour atteindre les autres objectifs mentionnés ci-dessus, il est par ailleurs prévu que le plateau selon l'invention se caractérise par le fait que chaque alvéole comprend au moins un organe élastique mobile entre une position de repos dans laquelle il se trouve lorsque ladite alvéole est vide et une position contrainte dans laquelle il se trouve lorsqu'une lame est logée dans ladite alvéole.

Dès lors, chaque lame introduite au sein d'une alvéole est bloquée en position par l'organe élastique, qui vient repousser ladite lame contre la paroi opposée à celle depuis laquelle il s'étend. En outre, l'orientation de cet organe élastique autorise un appui suffisant pour verrouiller une lame dans son alvéole, tout en autorisant aisément son insertion et son extraction, manuellement ou automatiquement.

Conformément à une variante de réalisation préférentielle, chaque organe élastique peut être constitué par au moins une languette ménagée en saillie par rapport au fond de ladite alvéole.

Il est par ailleurs prévu que ladite languette peut présenter une extrémité solidaire du fond de ladite alvéole et une extrémité opposée libre.

Dans ce cas, ladite languette s'étend depuis ledit fond de ladite alvéole de préférence de manière telle que son extrémité libre est orientée en direction de la paroi transversale.

Le plateau selon l'invention est en outre caractérisé par le fait qu'il est constitué au moins en partie en matériau plastique conférant des caractéristiques élastiques audit organe élastique.

Une caractéristique additionnelle du plateau selon l'invention est encore définie par le fait qu'au moins deux bords opposés de la plaque comportent chacun des moyens formant, selon le cas, un rail ou une coulisse destiné(e) à coopérer avec une coulisse ou un rail de forme complémentaire que comporte un meuble conformé pour héberger ledit plateau.

D'autres caractéristiques et avantages de l'invention ressortiront de la description détaillée qui va suivre d'un mode de réalisation non limitatif de l'invention, en référence aux figures annexées dans lesquelles :
- la figure 1 représente une vue en perspective de trois-quarts avant d'un plateau selon un mode préférentiel de réalisation de l'invention, montrant la répartition des alvéoles destinées à recevoir et maintenir les lames échantillonnées,
- la figure 2 est une vue agrandie d'une partie du plateau de la figure 1,
- la figure 3 représente une vue partielle du plateau de la figure 1 selon une coupe verticale effectuée le long de plusieurs alvéoles adjacentes, montrant la configuration et l'orientation d'une languette formant l'organe élastique au sein d'alvéoles vides et au sein d'alvéoles contenant une lame,
- la figure 4 représente schématiquement une vue en perspective d'un dispositif d'identification conçu pour recevoir un plateau selon l'invention,
- la figure 5 représente schématiquement une vue en perspective du dispositif de la figure 4, au sein duquel un plateau selon l'invention est introduit en vue de l'identification des lames que ledit plateau supporte,

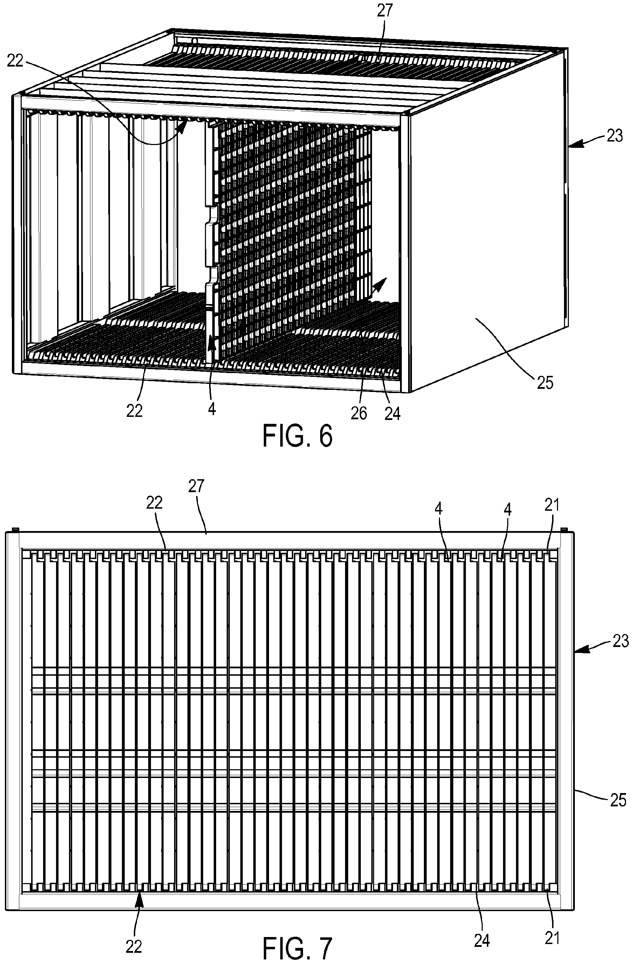

- la figure 6 illustre une vue partielle en perspective d'un meuble contenant un plateau selon l'invention, et
- la figure 7 est une vue de face du meuble de la figure 6.

La présente invention concerne le stockage et l'ordonnancement de lames échantillonnées 1.

Comme évoqué précédemment, lesdites lames 1 présentent une forme parallélépipédique rectangle. Pour rappel, de telles lames présentent des dimensions standardisées, de 75 à 76 mm de longueur, pour 25 à 26 mm de largeur, pour une épaisseur e d'environ 1 mm.

Lesdites lames 1 sont constituées en matériau transparent, notamment en verre. Elles reçoivent sur une portion d'une face, en particulier la face supérieure 2 recevant l'échantillon biologique, des informations d'identification, appliquées par exemple sur une étiquette adhésive qui y est apposée. La face inférieure 3, située à l'opposée, peut aussi recevoir des éléments, mais est généralement vierge, entièrement lisse.

On notera que lesdites informations d'identification peuvent être manuscrites mais se présentent le plus souvent sous forme de données encodées, notamment de type code à barres ou code matriciel. De telles informations d'identification permettent d'identifier de façon unique chaque lame 1, en lui attribuant notamment un numéro unique, la liant dans un système informatisé connexe, comme une base de données, à d'autres données notamment relatives au patient sur lequel a été prélevé ledit échantillon présent sur ladite lame 1.

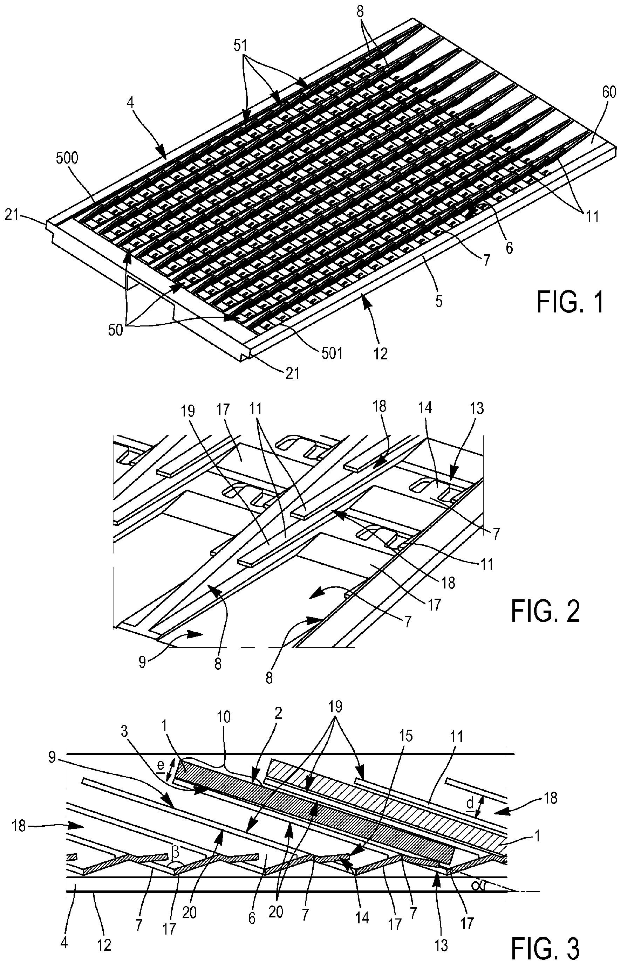

L'invention concerne un support de lames 1 se présentant sous forme d'un plateau 4. Comme visible sur la figure 1, un tel plateau 4 consiste en une plaque 5 de forme parallélépipédique rectangle. La plaque 5 s'étend selon un plan contenant ledit plateau 4, notamment la face inférieure de ce dernier. La plaque 5 comprend une pluralité d'alvéoles 6 alignées formant, dans la variante de réalisation illustrée, dix colonnes 50 et vingt rangées 51 parallèles. Chaque alvéole 6 est destinée à recevoir en insertion une lame 1. La position d'une lame 1 insérée au sein d'une alvéole 6 peut alors être référencée de façon bidimensionnelle, en relevant la colonne et la rangée correspondantes.

Afin d'autoriser l'insertion des lames 1, lesdites alvéoles 6 sont ménagées en creux au sein de l'épaisseur de ladite plaque 5. De plus, chaque alvéole 6 est conformée et dimensionnée au moins en partie complémentairement à la forme et aux dimensions d'une lame 1, au jeu près permettant d'assurer l'introduction et l'extraction de ladite lame 1 au sein et depuis ladite alvéole 6. En somme, chaque alvéole 6 présente une forme creuse globalement parallélépipédique rectangle, à savoir approximativement un pavé évidé.

Plus avant, chaque alvéole 6 est conformée pour permettre l'insertion d'une lame 1 selon un déplacement en translation, préférentiellement longitudinalement, à savoir selon la longueur de la lame 1. Chaque alvéole 6 est pourvue au moins d'un fond 7, de parois latérales opposées 8, et est accessible via une ouverture d'entrée 9. Dans la variante de réalisation illustrée, chaque alvéole 6 est prévue borgne, et comporte une paroi transversale 17 s'étendant entre les deux parois latérales opposées 8, en face de l'ouverture d'entrée 9. La paroi transversale 17 définit une butée d'arrêt pour une lame 1 insérée dans une alvéole 6. Selon le mode de réalisation préférentiel représenté sur les figures, la paroi transversale 17 forme un angle obtus β avec le fond 7 d'une alvéole 6. Selon un autre mode de réalisation, non représenté, ladite paroi transversale 17 peut être ménagée orthogonalement par rapport audit fond 7. Dès lors, lorsque la lame 1 est convenablement insérée dans l'alvéole 6, elle s'étend entre ses parois latérales 8 et est placée en butée contre sa paroi transversale 17.

Par ailleurs, chaque alvéole 6 comprend des moyens de guidage en translation d'une lame 1, au moins constitués par lesdites parois latérales 8. Selon le mode de réalisation représenté sur les figures 1-3, lesdits moyens de guidage comportent en outre des redents 11 ménagés en saillie au niveau de chacune des parois latérales 8. Ces redents 11 s'étendent depuis l'ouverture 9 de chaque alvéole 6 jusqu'à sa paroi transversale 17. Ils permettent de guider, selon un déplacement en translation, chaque lame 1 introduite ou extraite dans une alvéole 6 par coulissement ou glissement.

Il convient de noter que les redents 11 peuvent s'étendre sur 1 à 3 mm vers l'intérieur d'une alvéole 6. Chaque redent 11 peut s'étendre de manière continue ou discontinue le long d'une paroi latérale 8. Selon le mode continu représenté sur les figures, les redents 11 d'une première alvéole 6 forment alors, avec les redents 11 d'une alvéole 6 adjacente, une coulisse 18 de guidage d'une lame 1, assurant aussi une partie de son maintien au sein de son alvéole 6. En effet, chaque redent 11 d'une alvéole 6 est réalisé de façon espacée par rapport au redent 11 correspondant d'une alvéole adjacente 6, selon une distance d équivalente, au jeu près, à l'épaisseur e d'une lame 1. Cette dernière se retrouve donc bloquée orthogonalement, au niveau de ses bords destinés à coulisser, entre et le long de deux redents 11 partiellement superposés, ne pouvant que se déplacer en glissement selon la direction d'insertion et d'extraction, parallèlement aux redents 11.

Dans la variante de réalisation illustrée, comme visible sur la figure 3, les moyens de guidage peuvent aussi être constitués par une partie du fond 7 d'une alvéole 6. Dans cette variante de réalisation, la face supérieure 19 de chaque redent 11 vient en affleurement et dans la continuité du fond 7 d'une alvéole 6 adjacente. Une telle structure autorise chaque lame 1, lors de son insertion, à coulisser de façon continue non seulement entre les redents 11 de deux alvéoles 6 adjacentes, mais également entre un redent 11 et le fond 7 d'une alvéole 6 adjacente, jusqu'à son arrivée en butée contre la paroi transversale 17 d'une alvéole 6. En d'autres termes, la face supérieure 19 d'un redent 11 d'une première alvéole 6 vient en affleurement du fond 7 d'une alvéole adjacente 6. Ainsi, comme visible sur la figure 3, la face inférieure 20 de chaque redent 11 est en contact avec la face supérieure 2 d'une lame 1 insérée au sein d'une première alvéole 6, tandis que la face supérieure 19 d'un tel redent 11 est alors en contact avec la face inférieure 3 d'une autre lame 1 introduite dans l'alvéole 6 adjacente. Ainsi, les redents 11 de deux alvéoles 6 consécutives forment ensemble une coulisse 18 apte à recevoir les bords longitudinaux (les plus longs) d'une lame 1. D'autres configurations non représentées peuvent être envisagées, comme des alvéoles 6 s'étendant transversalement et recevant les lames 1 par coulissement le long de leurs bords latéraux d'extrémités (les plus courts).

De plus, afin d'autoriser l'insertion et l'extraction des lames 1 au sein des alvéoles 6, les fonds 7 desdites alvéoles 6 et les redents 11 formant les coulisses 18 sont inclinés par rapport au plan contenant ladite plaque 5. Tout d'abord, une telle inclinaison est prévue selon un angle α minimum autorisant et facilitant la préhension, manuelle ou automatique, de l'extrémité d'une lame 1 située au niveau de l'ouverture 9 d'une alvéole 6. D'autre part, cette inclinaison angulaire permet d'autoriser le recouvrement partiel de deux lames 1 insérées au sein d'alvéoles 6 adjacentes.

Un tel recouvrement s'effectue sur une partie de la longueur de chaque lame 1, laissant au moins découverte une portion 10 de la face supérieure 2 présentant les données d'identification. Cette configuration en recouvrement partiel est donc obtenue par l'inclinaison des coulisses 18 que comportent les alvéoles 6, combinée au fait que chaque coulisse 18 présente une longueur inférieure à celle d'une lame 1, créant un décalage et laissant dépasser une portion 10 de cette dernière hors de son alvéole 6.

En outre, comme évoqué précédemment, une partie d'une alvéole adjacente 6 est constituée par son fond 7 et par la surface des redents 11 d'une première alvéole 6. Cette configuration permet d'assurer le soutien de chaque lame 1 insérée dans son alvéole 6, de part et d'autre de ses faces inférieure 3 et supérieure 2, sur une distance suffisante. A titre d'exemple, cette distance suffisante peut représenter un tiers à trois-quarts de chaque lame 1. En d'autres termes, la longueur des redents 11 est déterminée pour que la course d'une lame 1 insérée au sein d'une alvéole 6 soit limitée de sorte qu'au moins sa portion 10 de face supérieure 2 dépasse de l'alvéole 6. De préférence, la portion 10 de face supérieure 2 correspond à au moins la surface occupée par les données d'identification de chaque lame 1. Cette course sur une distance donnée est arrêtée par la paroi transversale 17 formant butée.

Ainsi, c'est bien l'inclinaison combinée à la longueur réduite de chaque fente 10, par rapport à la longueur totale d'une lame 1, qui permet de positionner les lames 1 en recouvrement partiel, produisant un décalage laissant apparente la portion 10 de face 2 où sont situées les données d'identification.

Avantageusement, l'inclinaison du fond 7 des alvéoles 6 et des redents 11 présente un angle α minimum de 5° et un angle α maximum de 45°, par rapport au plan dans lequel s'étend le plateau 4. Au-delà de 45° d'inclinaison, ledit plateau 4 présenterait une hauteur conséquente et s'apparenterait plutôt à un tiroir.

Préférentiellement, l'angle α est compris entre 8° et 28°. Ce choix permet d'optimiser, d'une part, le nombre d'alvéoles 6 pour une longueur de plateau 4 donnée, mais aussi d'assurer une inclinaison suffisante, tout en conservant une épaisseur raisonnable dudit plateau 4.

On notera que l'inclinaison est déterminée par rapport au plan contenant ledit plateau 4, à savoir le plan coïncidant avec sa face inférieure, ou encore sa face supérieure. Dès lors, cette inclinaison est indépendante de l'orientation du plateau 4 lui-même, qui peut être horizontale, verticale ou bien en biais.

Avantageusement, la configuration inclinée et en recouvrement partiel des lames 1, maintenues transversalement par les redents 10 et le fond 7 des alvéoles 6 au niveau de leurs bords, en n'autorisant que le déplacement en coulissement au sein des coulisses 18, confère auxdites lames 1 un maintien accru, même en cas de retournement ou renversement dudit plateau 4, à l'exception d'un retournement selon la direction de coulissement au sein des alvéoles 6, dans le sens d'extraction, limitant ainsi considérablement les risques.

Avantageusement, afin d'empêcher un tel déplacement dans le sens d'extraction, chaque alvéole 6 comprend des moyens de maintien 13 en insertion d'une lame 1.

De tels moyens de maintien 13 comportent au moins un organe 14 prévu élastique d'appui contre une face de ladite lame 1 en insertion au sein de ladite alvéole 6. En d'autres termes, au moins un organe élastique 14 est présent dans chaque alvéole 6 et vient au contact d'une face de la lame 1 qui y est introduite, pour venir la plaquer contre une paroi ou un élément de ladite alvéole 6, notamment les moyens de guidage, en particulier les redents 11.

De plus, le caractère élastique de l'organe 14 assure sa déformation, notamment lors de l'insertion de la lame 1, tandis que sa résilience assure une pression suffisante pour empêcher la lame 1 de bouger, tout en autorisant le retour de cet organe 14 en position non contrainte une fois la lame 1 extraite de son alvéole 6.

Ainsi, une lame 1 est introduite en force dans une alvéole 6, pour déformer et repousser l'organe 14, jusqu'à arriver en butée au niveau de la paroi transversale 17 de l'alvéole 6. Ladite lame 1 se retrouve verrouillée, ne pouvant plus se déplacer librement dans le sens d'extraction.

Selon le mode préférentiel de réalisation, chaque organe 14 peut être constitué par au moins une languette 14 ménagée en saillie par rapport au fond 7 de ladite alvéole 6. En d'autres termes, chaque languette 14 s'étend vers l'intérieur d'une alvéole 6. Dans la variante de réalisation illustrée sur les figures, chaque organe 14 se présente sous forme d'une seule languette 14 par alvéole 6.

De façon subsidiaire, une extrémité de ladite languette 14 peut être solidaire dudit fond 7 tandis qu'une extrémité opposée 15 peut demeurer libre. Cette languette 14 peut donc être constituée par le prolongement du fond 7 et du matériau le constituant, au travers d'une découpe ménagée dans ledit fond 7 et entourant chaque languette 14.

Ladite languette 14 peut s'étendre par rapport audit fond 7 de manière telle que son extrémité libre soit orientée en direction de la paroi transversale 17 de l'alvéole 6 considérée.

Enfin, comme évoqué précédemment, ledit plateau 4 peut être constitué au moins en partie en matériau plastique conférant des caractéristiques élastiques aux moyens de maintien des lames 1, en particulier aux languettes 14.

Ainsi, les moyens de maintien sous forme d'un organe 14 élastique, de préférence une languette saillante 14, viennent en appui contre la face inférieure 3 de la lame 1 insérée dans son alvéole 6, y assurant son parfait maintien, sans risque qu'elle en sorte naturellement, son extraction nécessitant une opération manuelle ou automatique volontaire.

En outre, l'inclinaison de cette languette 14 par rapport au fond 7, ses dimensions et les caractéristiques élastiques de son matériau, assurent une force de maintien suffisante, sans risquer d'endommager la lame 1 ainsi contrainte et bloquée par ces moyens de maintien au sein de chaque alvéole 6 d'un tel plateau 4.

Il convient encore de noter que selon une caractéristique additionnelle de l'invention, les deux bords longitudinaux opposés 500, 501 du plateau 4 selon l'invention illustré aux figures sont prolongés chacun par un rail 21 destiné à coopérer avec une coulisse 22 de forme complémentaire que comporte un meuble 23 conformé pour héberger au moins un tel plateau 4.

Un tel meuble 23 conformé pour héberger au moins un plateau 4 comporte un fond 24 surmonté par une paroi périphérique 25 présentant une ouverture d'entrée 26 et une paroi supérieure 27 opposée audit fond 24. Dans la variante de réalisation illustrée, le meuble 23 comporte des moyens formant une coulisse 22 s'étendant de manière appropriée sur les faces internes de son fond 24 et de la paroi supérieure 27. Une telle structure permet de stocker les plateaux 4 verticalement dans le meuble 23, et ainsi d'éviter d'éventuels phénomènes de déformation observés lorsqu'ils sont stockés horizontalement. Il a également été constaté qu'un stockage vertical des plateaux 4 permet d'optimiser la capacité d'accueil du meuble 23 et d'y loger davantage de plateaux 4 qu'en situation de stockage à l'horizontale. Ainsi, davantage de lames 1 peuvent être conservées dans un même volume.

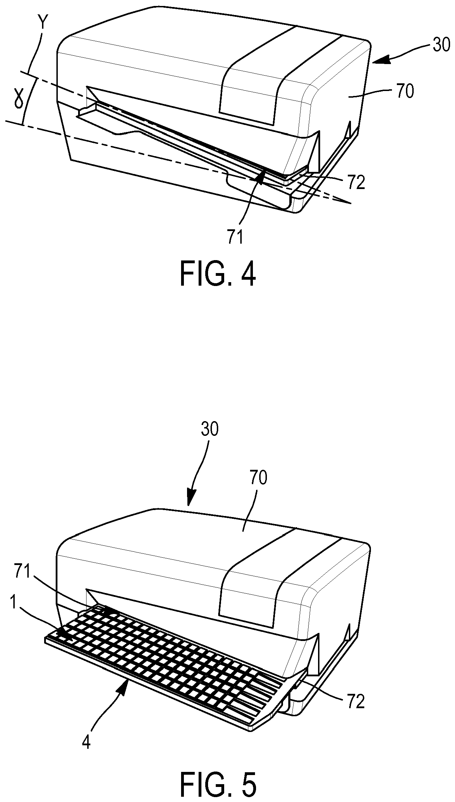

Les figures 4 et 5 illustrent un dispositif 30 d'identification de lames 1 contenues au sein d'un plateau 4 selon l'invention, tel que précédemment décrit.

Un tel dispositif 30 est constitué d'une enveloppe 70 enfermant des moyens conçus pour détecter la présence d'une lame 1 au sein de chaque alvéole 6 et pour identifier une lame 1 présente, à savoir détectée. Cette identification s'opère au travers de la lecture des données d'identification apposées sur la portion 10 de la face supérieure 2 de chaque lame 1 qui demeure apparente du fait du recouvrement partiel conféré par la configuration spécifique des alvéoles 6 du plateau 4 selon l'invention.

De plus, l'enveloppe 70 comporte une ouverture 71 prévue traversante, dimensionnée complémentairement aux dimensions dudit plateau 4, au travers de laquelle il peut être introduit et extrait au sein du et depuis une cavité 72 formée dans ledit dispositif 30.

Avantageusement, comme visible sur le mode de réalisation préférentiel du dispositif 30 représenté aux figures 4 et 5, l'ouverture 71 et la cavité 72 s'étendent longitudinalement, à savoir selon le bord le plus long dudit dispositif 30. De plus, la cavité 72 est inclinée par rapport à un plan horizontal contenant le dispositif 1. En particulier, l'axe longitudinal Y de la cavité 72 s'étend en biais, et est incliné d'un angle γ par rapport au plan formé par la surface inférieure du dispositif 30, qui correspond de façon non limitative le plan horizontal sur lequel il est destiné à être placé.

A l'instar du plateau 4, cette inclinaison permet d'optimiser les dimensions du dispositif 30, limitant son encombrement, en particulier sa longueur. En contrepartie, le dispositif 30 présente alors une hauteur plus importante. Cette optimisation permet de positionner plus aisément ledit dispositif 30 sur des meubles d'une largeur limitée.

De façon connexe, l'inclinaison du plateau 4 au sein du dispositif 30 permet d'améliorer la détection et la lecture par les moyens prévus à cet effet.

Selon un mode préférentiel de réalisation, l'angle d'inclinaison γ de l'axe longitudinal Y de la cavité 72 est d'au moins 5° et au maximum de 90°. De préférence, cet angle d'inclinaison γ est compris entre 5° et 45°, par exemple égal à 8°.

On notera que le choix des angles au sein du plateau 4 comme du dispositif 30 n'est aucunement arbitraire, mais effectué dans le but d'optimiser leur encombrement respectif.

## Revendications

1. Plateau (4) destiné au stockage de lames d'échantillonnage (1), constitué d'une plaque (5) comprenant une pluralité d'alvéoles (6) alignées, ménagées en creux au sein de l'épaisseur de ladite plaque (5), selon une forme et des dimensions conçues aptes à épouser au moins partiellement lesdites lames (1), chaque alvéole (6) étant au moins constituée par un fond (7) et deux parois latérales opposées (8), présentant une ouverture d'entrée (9) et étant au moins en partie fermée par une paroi transversale (17) s'étendant entre les parois latérales opposées (8), en face de ladite ouverture d'entrée (9), chaque alvéole (6) comprenant au moins deux redents (11) respectivement ménagés en saillie au niveau de chacune des parois latérales (8), lesdits redents (11) s'étendant depuis l'ouverture d'entrée (9) jusqu'à la paroi transversale (17), parallèlement au fond (7) et formant avec les redents (11) de l'alvéole (6) adjacente des coulisses (18) destinées à permettre un guidage en translation des bords d'une lame (1) introduite dans une alvéole (6), **caractérisé par le fait que** le fond (7) de chaque alvéole (6) est prévu incliné d'un angle α par rapport au plan dudit plateau (4) tandis que la longueur de chaque coulisse (18) est inférieure à la longueur d'une lame (1) destinée à être stockée dans une alvéole (6).

2. Plateau (4) selon la revendication 1, **caractérisé par le fait que** l'angle d'inclinaison α du fond (7) de chaque alvéole (6) est compris entre 5° et 45°.

3. Plateau (4) selon la revendication 2, **caractérisé par le fait que** l'angle d'inclinaison α du fond (7) de chaque alvéole (6) est compris entre 8° et 25°.

4. Plateau (4) selon l'une quelconque des revendications précédentes, **caractérisé par le fait que** chaque alvéole (6) comprend au moins un organe élastique (14) mobile entre une position de repos dans laquelle il se trouve lorsque ladite alvéole (6) est vide et une position contrainte dans laquelle il se trouve lorsqu'une lame (1) est logée dans ladite alvéole (6) .

5. Plateau (4) selon la revendication 4, **caractérisé par le fait que** chaque organe élastique (14) est constitué par au moins une languette (14) ménagée en saillie par rapport au fond (7) de ladite alvéole (6).

6. Plateau (4) selon la revendication 5, **caractérisé par le fait que** ladite languette (14) présente une extrémité solidaire du fond (7) de ladite alvéole (6) et une extrémité opposée (15) libre.

7. Plateau (4) selon la revendication 6, **caractérisé par le fait que** ladite languette (14) s'étend depuis ledit fond (7) de ladite alvéole (6) de manière telle que son extrémité libre (15) est orientée en direction de la paroi transversale (17).

8. Plateau (4) selon l'une quelconque des revendications 4 à 7, **caractérisé par le fait qu'**il est constitué au moins en partie en matériau plastique conférant des caractéristiques élastiques audit organe élastique (14).

9. Plateau (4) selon l'une quelconque des revendications précédentes, **caractérisé par le fait qu'**au moins deux bords opposés (500, 501) de la plaque (5) comportent chacun des moyens formant, selon le cas, un rail (21) ou une coulisse destiné (e) à coopérer avec une coulisse (22) ou un rail de forme complémentaire que comporte un meuble (23) conformé pour héberger ledit plateau (4).

## Patentansprüche

1. Tablett (4), das zum Lagern von Proben-Objektträgern (1) bestimmt ist, bestehend aus einer Platte (5), umfassend eine Vielzahl ausgerichteter Fächer (6), die konkav in die Stärke der Platte (5) eingearbeitet sind, gemäß einer Form und Abmessungen, die so gestaltet sind, dass sie imstande sind, sich mindestens teilweise an den Objektträgern (1) anzuliegen, wobei jedes Fach (6) aus mindestens einem Boden (7) und zwei gegenüberliegenden Seitenwänden (8) besteht, aufweisend eine Eingangsöffnung (9), und dabei mindestens zum Teil von einer transversalen Wand (17) verschlossen ist, die sich zwischen den gegenüberliegenden Seitenwänden (8) erstreckt, gegenüber der Eingangsöffnung (9), wobei jedes Fach (6) mindestens zwei Stufen (11) umfasst, die jeweils hervorstehend im Bereich jeder der Seitenwände (8) ausgebildet sind, wobei sich die Stufen (11) ab der Eingangsöffnung (9) bis zu der transversalen Wand (17) erstrecken, parallel zum Boden (7), und mit den Stufen (11) des benachbarten Fachs (6) Gleitführungen (18) bilden, die bestimmt sind, eine translatorische Führung der Ränder eines in ein Fach (6) eingeführten Objektträgers (1) zu erlauben, **dadurch gekennzeichnet, dass** der Boden (7) jedes Fachs (6) in einem Winkel α in Bezug auf die Ebene des Tablett (4) geneigt vorgesehen ist, wogegen die Länge jeder Gleitführung (18) kleiner als die Länge eines Objektträgers (1) ist, der bestimmt ist, in einem Fach (6) gelagert zu sein.

2. Tablett (4) nach Anspruch 1, **dadurch gekennzeichnet, dass** der Neigungswinkel α des Bodens (7) jedes Fachs (6) zwischen 5° und 45° liegt.

3. Tablett (4) nach Anspruch 2, **dadurch gekennzeichnet, dass** der Neigungswinkel α des Bodens (7) jedes Fachs (6) zwischen 8° und 25° liegt.

4. Tablett (4) nach einem der vorangehenden Ansprüche, **dadurch gekennzeichnet, dass** jedes Fach (6) mindestens ein elastisches Organ (14) umfasst, das zwischen einer Ruhestellung, in welcher es sich befindet, wenn das Fach (6) leer ist, und einer gespannten Stellung, in welcher es sich befindet, wenn ein Objektträger (1) in dem Fach (6) untergebracht ist, beweglich ist.

5. Tablett (4) nach Anspruch 4, **dadurch gekennzeichnet, dass** jedes elastische Organ (14) aus mindestens einer Zunge (14) besteht, die in Bezug auf den Boden (7) des Fachs (6) hervorstehend ausgebildet ist.

6. Tablett (4) nach Anspruch 5, **dadurch gekennzeichnet, dass** die Zunge (14) ein Ende aufweist, das mit dem Boden (7) des Fachs (6) fest verbunden ist, und ein freies gegenüberliegendes Ende (15).

7. Tablett (4) nach Anspruch 6, **dadurch gekennzeichnet, dass** sich die Zunge (14) ab dem Boden (7) des Fachs (6) derart erstreckt, dass ihr freies Ende (15) in Richtung der transversalen Wand (17) zeigt.

8. Tablett (4) nach einem der Ansprüche 4 bis 7, **dadurch gekennzeichnet, dass** es mindestens zum Teil aus Kunststoff besteht, der dem elastischen Organ (14) elastische Eigenschaften verleiht.

9. Tablett (4) nach einem der vorangehenden Ansprüche, **dadurch gekennzeichnet, dass** mindestens zwei gegenüberliegende Ränder (500, 501) der Platte (5) jeweils Mittel aufweisen, die je nach Fall eine Schiene (21) oder eine Gleitführung bilden, die bestimmt ist, mit einer Gleitführung (22) oder einer Schiene komplementärer Form zusammenzuwirken, die ein Möbel (23) aufweist, das ausgebildet ist, um das Tablett (4) aufzunehmen.

## Claims

1. Tray (4) for sampling (1), consisting of a plurality of aligned cells (6), slide plate storage (5) comprising recesses within the thickness of the plate (5) of shape and dimensions designed to fit the slides (1), wherein each cell (6) is at least partially constituted by a bottom (7) and two opposite side walls (8) having an inlet opening (9) and being at least partly closed by a transverse wall (17) extending between the opposite side walls (8) and opposite the inlet opening (9), wherein each cell (6) comprises at least two redents (11) respectively protruding from each of the side walls (8), wherein the redents (11) extend from the inlet opening (9) to the transverse wall (17), parallel to the bottom (7) and forming with the redents (11) of the cell (6) for adjacent slots (18) to allow guidance in translation of the edges of a slide (1) introduced into a cell (6), **characterized in that** the bottom (7) of each cell (6) is inclined at an angle with respect to the plane of the tray (4) while the length of each slot (18) is less than the length of a slide (1) to be stored in a cell (6).

2. Tray (4) according to claim 1, **characterized in that** the angle of inclination of the bottom (7) of each cell (6) is between 5° and 45°.

3. Tray (4) according to claim 2, **characterized in that** the angle of inclination of the bottom (7) of each cell (6) is between 8° and 25°.

4. Tray (4) according to any one of the preceding claims, **characterized in that** each cell (6) comprises at least one elastic member (14) that is movable between a position of rest in which it is located when the cell (6) is empty and a constrained position when a blade (1) is housed in the cell (6).

5. Tray (4) according to claim 4, **characterized in that** each elastic member (14) is constituted by at least one tongue (14) projecting from the bottom (7) of the cell (6).

6. Tray (4) according to claim 5, **characterized in that** the tongue (14) has an end secured to the bottom (7) of the cell (6) while an opposite end (15) is free.

7. Tray (4) according to claim 6, **characterized in that** the tongue (14) extends from the bottom (7) of the cell (6) so that its free end (15) is oriented in the direction of the transverse wall (17).

8. Tray (4) according to any one of claims 4 to 7, **characterized in that** it consists at least in part of plastic material imparting elastic characteristics to the elastic member (14).

9. Tray (4) according to any one of the preceding claims, **characterized in that** at least two opposite edges (500, 501) of the plate (5) each comprise means forming, as appropriate, a rail (21) or a slide intended (e) to cooperate with a slot (22) or a rail of complementary shape that comprised in a piece of furniture (23) that is shaped to accommodate the plate (4).
